(19) Europäisches Patentamt

European Patent Office

Office européen des brevets

(11) **EP 0 866 715 B1**

(12) **FASCICULE DE BREVET EUROPEEN**

(45) Date de publication et mention
de la délivrance du brevet:
**17.07.2002 Bulletin 2002/29**

(21) Numéro de dépôt: **96939117.6**

(22) Date de dépôt: **14.11.1996**

(51) Int Cl.[7]: **A61K 35/78**, A61K 7/06,
A61P 17/00

(86) Numéro de dépôt international:
**PCT/FR96/01796**

(87) Numéro de publication internationale:
**WO 97/18823 (29.05.1997 Gazette 1997/23)**

(54) **ASSOCIATION EXTRAIT DE MYRTE ET ANTIFONGIQUES**

KOMBINATION EINES MYRTE-EXTRAKTES MIT EINEM FUNGIZID

COMBINATION OF A MYRTLE EXTRACT AND ANTIFUNGAL AGENTS

(84) Etats contractants désignés:
**AT BE CH DE DK ES FI FR GB GR IE IT LI LU MC NL PT SE**

(30) Priorité: **17.11.1995 FR 9513640**

(43) Date de publication de la demande:
**30.09.1998 Bulletin 1998/40**

(73) Titulaire: **Pierre Fabre Dermo-Cosmetique
92100 Boulogne-Billancourt (FR)**

(72) Inventeurs:
• **LAGARDE, Isabelle
F-31520 Ramonville-Saint-Agne (FR)**
• **JEANJEAN, Michel
F-31320 Castanet-Tolosan (FR)**
• **FABRE, Bernard
F-31450 Belberaud (FR)**

(74) Mandataire: **Ahner, Francis
Cabinet Régimbeau
20, rue de Chazelles
75847 Paris cedex 17 (FR)**

(56) Documents cités:
**WO-A-96/40180          DE-A- 4 401 274
FR-A- 2 296 401**

• BULL. FAC. PHARM. (CAIRO UNIVERSITY), vol.
27, no. 1, 1989, pages 101-103, XP000577840
G.A. EL HOSSARY: "Phytochemical study of the
leaves of Myrtus communis L. grown in Egypt"
• J. ETHNOPHARMACOL., vol. 13, no. 2-3, 1986,
pages 167-174, XP002010595 F. TAMMARO:
"Plants used in phytotherapy, cosmetics and
dying in the Pramanda district (Epirus,
North-West Greece)"
• G. GARNIER ET AL.: "Ressources Médicinales
de la flore française, Tome II" 1961 , VIGOT
FRERES , PARIS XP002010596 voir page 848 -
page 850

## Description

La présente invention se rapporte à des compositions utiles en dermatologie et/ou en cosmétologie et qui possèdent une activité antimicrobienne et plus particulièrement antifongique.

Les antifongiques classiques sont des agents chimiques qui s'opposent à la prolifération des micro-organismes. On peut citer par exemple les dérivés des N-hydroxypyridones, le sel de Zinc du pyridine 1 oxy 2 thiol, le sulfure de sélénium, les conservateurs à spectre antifongiquc tels que l'acide sorbique, l'acide déhydroacétique d'Octopyrox® et les tensio-actifs dérivés undécyléniques.

Toutefois, parmi les molécules citées, certaines ont un potentiel cytotoxique in vitro, il est donc souhaitable d'abaisser les concentrations nécessaires à l'activité afin de diminuer la cytotoxicité, voire de formuler avec des molécules protégeant l'intégrité des cellules cutanées plus ou moins lésées et enflammées par la présence d'un germe pathogène.

Comme indiqué dans sa demande de brevet français antérieure FR-A-2735026 (n⁻ 9506695), et dans la demande de brevet internationale WO 96/40180 la Demanderesse a mis en évidence qu'un extrait de Myrtus communis possédait de manière inattendue une activité antifongique intrinsèque.

Des études microbiologiques complémentaires ont montré que l'extrait de *Myrtus* est capable de potentialiser l'activité antifongique d'autres molécules d'activité similaire.

C'est pourquoi la présente invention a pour objet une composition dermatologique et/ou cosmétologique caractérisée en ce qu'elle contient un extrait de Myrte associé à au moins un antifongique actif sur l'agent responsable des pellicules : le Pityrosporum ovale.

Ces antifongiques seront avantageusement choisis parmi :

(i) les conservateurs dont le spectre est orienté vers les fongi et particulièrement les levures, tels que l'acide sorbique et l'acide déhydroacétique ;
(ii) les actifs répertoriés comme agents antilevures, tels que la zinc pyrithione et la piroctone-olamine, et
(iii) des tensio-actifs dérivés de l'acide undécylénique, en particulier l'undécylényl sulfosuccinate de sodium ou le monoéthanolamide.

En ce qui concerne la plante, on rappellera tout d'abord que le Myrte, Myrtus communis, appartient à la famille des Myrtacées. C'est un arbuste qui peut atteindre 2 à 3 mètres de haut, ses tiges sont irrégulières, sont recouvertes d'une écorce rousse presque lisse. Les feuilles sont opposées, persistantes, coriaces, vert foncé. Le limbe est ovale, aigu au sommet, parsemé de ponctuations glanduleuses translucides, il s'atténue en un très court pétiole.

Les fleurs sont blanches, odorantes et solitaires, portées sur un long pédoncule à l'aisselle des feuilles. Elles s'épanouissent de mai à juillet. Le calice est soudé à l'ovaire dans sa partie inférieure. Il présente 5 divisions triangulaires, étalées, plus courtes que les pétales. La corolle a 5 pétales libres entre eux. Les étamines sont libres et nombreuses. L'ovaire est trioculaire et surmonté d'un style terminé par un stigmate unique. Les 3 loges de l'ovaire renferment chacune de nombreux ovules. Le fruit est une baie charnue, ovoïde, noir bleuâtre à maturité. Il est surmonté des 5 dents persistantes du calice. Il renferme un grand nombre de graines dépourvues d'albumen.

Le Myrte croit indifféremment en terrains calcaires ou silicieux. Il craint le gel et ne s'élève donc pas à une grande altitude. Il est très commun en France sur le littoral méditerranéen, surtout en Provence.

Il habite également l'Europe méridionale, les régions occidentales et méridionales de l'Asie, le Nord de l'Afrique. Le Myrte est surtout utilisé pour sa richesse en huile essentielle présente dans les feuilles mais aussi dans le fruit. Cette essence s'extrait par distillation à la vapeur ou par extraction avec des solvants apolaires comme l'hexane, est de couleur verdâtre et d'odeur agréable. Elle est composée d'eucalyptol, de linalol, géraniol, pinène et limonène et de substances plus spécifiques comme le myrtol et le myrténol.

Les feuilles renferment également de fortes proportions de tanins, galliques en majorité, 14 % en moyenne, des flavonoïdes (myricétol, kaempferol et leurs dérivés glycosilés), des coumarines (aesculine, aesculétine) et des acides phénoliques.

Les fruits, en plus des huiles essentielles, contiennent une grande quantité de composés polyphénoliques dont une partie est constituée de tanins hydrolysables et condensés. On trouve également des composés phénoliques plus simples comme la quercétine, la patulétine, les acides galliques et ellagiques.

L'extraction de ce végétal peut être mise en oeuvre par différents processus classiques bien connus de l'homme du métier.

Les feuilles et/ou les fruits sont broyés puis extraits par un alcool de $C_1$ à $C_4$ ou par un mélange eau-alcool de $C_1$ à $C_4$ ou eau-acétone dans des proportions variant de 100-0 à 0-100.

Le rapport plante/solvant varie de 1 à 4 à 1 à 20. L'extraction peut se faire sous agitation ou statiquement. Les températures d'extractions varient de la température ambiante à l'ébullition du solvant d'extraction. La durée d'extraction se situe entre 1 heure et 24 heures.

Une fois l'extraction effectuée, les solutions sont récupérées par filtration ou par essorage. Une seconde extraction reprenant les mêmes paramètres que la première peut être effectuée. Les 2 solutions sont alors jointes.

Les solutions sont évaporées sous pression réduite à des températures situées entre 40°C et 100° C jusqu'à obtention d'une poudre. On peut également lors de l'opération de concentration, ajouter un solvant à haut point d'ébullition comme la glycérine, le propylène glycol, le butylène glycol ou le transcutol.

Dans ce cas, l'évaporation est menée jusqu'à évaporation complète de l'alcool de $C_1$ à $C_4$ et de l'acétone et jusqu'à des proportions eau-solvant de haut point d'ébullition variant de 0-100 à 80-20. Les teneurs en matière sèche de ces extraits liquides varient de 15 à 1 %. Ces extraits sont filtrés si nécessaire.

Les différents extraits de Myrte sont dosés pour leur teneur en polyphénols par dosage colorimétrique avec le réactif de Folin Ciocalcieu. Les teneurs varient de 5 à 40 % par rapport à la matière sèche de l'extrait.

La teneur en flavonoïdes totaux est dosée par absorption dans l'ultra-violet à 385 nm après réaction avec une solution alcoolique de chlorure d'aluminium. Les teneurs en flavonoïdes varient de 0,5 à 5 %.

On indiquera ci-après à titre d'exemples non limitatifs différents modes de mise en oeuvre d'extraction de Myrte.

**Exemple 1 :**

100 kg de feuilles et fruits secs de Myrte sont broyés puis extraits par 700 kg d'éthanol à 50%. L'extraction est réalisée sous reflux pendant 1 heure et sous agitation. Les solutions sont récupérées après filtration, puis concentrées sous pression réduite à 60 ° C. Lors de la concentration, on rajoute 200 kg de propylène glycol, la concentration est menée jusqu'à l'alimentation d'une solution pesant 220 kg. La teneur en matière sèche se situe entre 8 et 12 %, celle des polyphénols par rapport à la matière sèche entre 20 et 40 %, celle des flavonoïdes par rapport à la matière sèche, entre 1,5 et 3,5 %.

**Exemple 2 :**

1 kg de feuilles sèches, broyées, est extrait par 10 kg de méthanol pendant 12 h. La solution méthalonique est concentrée sous vide à 60 °C, puis séchée. L'extrait sec est broyé. La poudre titre entre 30 et 40 % en polyphénols et entre 3 et 4 % de flavonoïdes.

**Exemple 3 :**

10 kg de feuilles et fruits broyés sont extraits par un mélange eau-éthanol -80-20 sous reflux et sous agitation pendant 3 heures. La solution obtenue est essorée puis filtrée. Sa teneur en polyphénols par rapport à la matière sèche se situe entre 5 et 10 %. Les flavonoïdes varient de 0,5 à 1,5 %.

Les compositions selon la présente invention contiennent de préférence des associations binaires d'extrait de Myrte avec un antifongique.

Selon un aspect de l'invention, les compositions antifongiques à usage dermocosmétique contiennent au moins une association synergique d'extrait de Myrtus et d'un sel de N hydroxypiridone et plus particulièrement le sel d'éthanolamine de l'Octopirox® (piroctone olamine).

Des associations particulièrement avantageuses sont obtenues pour des associations dans lesquelles le rapport extrait de Myrte / sel d'Octopyrox® est compris entre 1 et 20. De préférence, le sel de N hydroxypyridone est un sel d'éthanolamine.

Selon un autre aspect, les compositions selon l'invention contiennent une association synergique d'extrait de Myrtus et d'un sel de pyrithione, comme par exemple le zinc pyrithione.

On utilisera notamment ce sel avec un rapport des concentrations Extrait de Myrte/ zinc pyrithione compris entre 1 et 1000.

Enfin, certains excipients présentent des activités antifongiques intrinsèques tels les tensio-actifs dérivés de l'acide undécylénique.

Les rapports des concentrations optimaux entre l'extrait de Myrtus et l'undécylényl sulfosuccinate de sodium sont de préférence compris entre 1 et 3.

De plus, les excipients actifs formulés à leur valeur d'excipient c'est-à-dire de l'ordre de 2 à 5 % présentent des propriétés naturellement fongicides avec des cinétiques d'activité rapides. Ainsi, on obtient non seulement une synergie par le jeu de l'optimisation des rapports de concentration comme précédemment décrite, mais une potentialisation en terme de vitesse d'efficacité se rapprochant de la fongicidie.

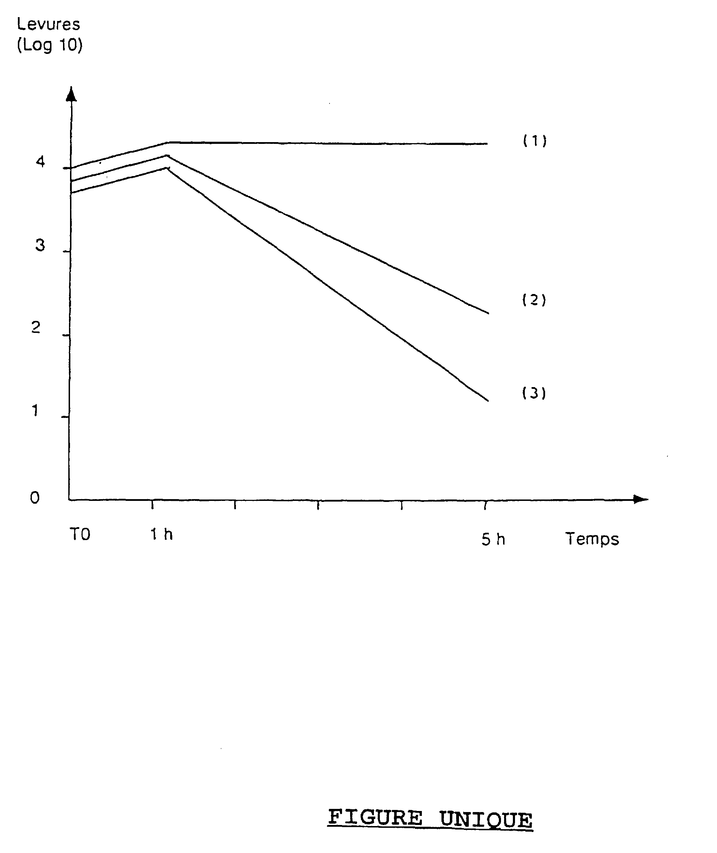

En effet, le temps mis pour faire chuter la population de P.ovale d'un Logarithme décimal ou Decimal Value (D.Value) est diminué de moitié lorsque 0,3 % d'extrait de Myrte et 2 % d'undécylényl sulfosuccinate de sodium sont associés comme illustré à la figure unique annexée.

Ces activités ont été testées sur la levure Pityrosporum ovale (souche sauvage obtenue en milieu hospitalier - CHU Toulouse Rangueil, France).

La mesure de l'activité des associations a été effectuée par le calcul de l'index FIC.

La synergie peut être mise en évidence par le calcul des index FIC (Fractional Inhibitory Concentration) des produits. Pour cela, on mesure la valeur de la CMF (ou Concentration Minimale Fongistatique), des principes actifs à tester vis-à-vis du germe.

Le FIC d'un produit A est défini comme :

$$FIC_A = \frac{\text{CMF du produit A en association}}{\text{CMF du produit A seul}}$$

L'association de deux produits A et B est synergique si la somme FIC des rapports ($FIC_A + FIC_B$) est inférieure ou égale à 0,75.

Plus la valeur FIC est faible, plus la synergie est importante.

On considère qu'il y a simple additivité pour des valeurs de FIC comprises entre 0,75 et 1, 1 et indifférence dans l'intervalle compris entre 1,1 et 2 ; au-delà, l'association est notée antagoniste.

Par exemple, l'undécylsulfosuccinate de sodium nécessite une concentration moyenne de 0,01 % au lieu de 0,02 % (gain de 100 %) et il ne faut que 0,02 % d'extrait de Myrte au lieu de 0,04 % (gain de 100 %).

Ces résultats confirment l'intérêt d'associer l'extrait de Myrte aux différents antifongiques cités:

**Exemples de FIC Index** :

| Produit | Seul | Associé | FIC | FIC Index |
|---|---|---|---|---|
| Extrait Myrte | 0,04 % | 0,02 % | 0,5 | |
| Undécyl sulfocuccinate de sodium | 0,02 % | 0,01 % | 0,25 | 0,75 |

| Produit | Seul | Associé | FIC | FIC Index |
|---|---|---|---|---|
| Extrait Myrte | 0,04% | 0,02% | 0,5 | 0,75 |
| Piroctone olamine | 0,005% | 0,0012% | 0,25 | |

| Produit | Seul | Associé | FIC | FIC Index |
|---|---|---|---|---|
| Extrait Myrte | 0,04 % | 0,01 % | 0,23 | 0,48 |
| Zn pirithione | 0,00025% | 0,00006% | 0,25 | |

D'autres molécules antifongiques associées à l'extrait de Myrte sont susceptibles de présenter un comportement synergique, par exemple, certains conservateurs, les dérivés imidazolés.

Le matériel et la méthode utilisés pour conduire cette expérimentation sont mentionnés ci-après :

**Matériel :**

Souche : Pityrosporum ovale (sauvage)
Milieu de culture : Bouillon et gélose Dixon
Neutralisant : Bouillon Trypticase soja supplémenté avec 10 % de Tween 80.

Des dilutions sériées en bouillon Dixon supplémenté par une gamme d'antifongique permettant d'obtenir la Concentration Minimale Fongistatique (CMF) de l'antimicrobien vis-à-vis du germe-test et ceci après 72 h de culture à 32°C.

Le croisement d'une gamme de deux produits centrés au niveau des deux CMF permet d'obtenir une nouvelle CMF des deux produits associés. La méthode décrite est la méthode de l'échiquier.

Les produits sont préparés extemporanément dans différents excipients pour lesquels la non activité antifongique a été vérifiée au préalable.

- piroctone-olamine = solution aqueuse à 0,05 %
- pyrithione zinc = solution acqueuse au 1/100 de la solution mère à 0,5 %
- sulfosuccinate sodique de l'acide undécylénique = solution aqueuse à 5 %.

On mentionnera ci-après quelques exemples de formulations des compositions capillaires conformes à la présente invention.

| Exemple A : SHAMPOOING | |
|---|---|
| EXTRAIT DE MYRTE | 5 g |
| ACIDE SALICYLIQUE | l g |
| ALKYLETHER SULFATE DE SODIUM | 10 g |
| COCAMIDOPROPYL BETAINE | 3 g |
| DIETHANOLAMIDE D'ACIDES GRAS | l g |
| ESSENCE DE GIROFLE | 0,05 g |
| PIROCTONE-OLAMINE | 0,80 g |
| LAURAMINE OXYDE | 0,3 g |
| MYRISTAMINE OXYDE | 0,5 g |
| EAU PURIFIEE QSP | 100 ml |

| Exemple B : SHAMPOOING | |
|---|---|
| EXTRAIT DE MYRTE | 3 g |
| UNDECYLENYL SULFOSUCCINATE DE SODIUM | 0,5 à 2 g |
| ACIDE SALICYLIQUF, | 1 g |
| ALKYLETHER SULFATE DE SODIUM | 10 g |
| ALKYL POLYGLUCOSIDE | 4 g |
| COCAMIDOPROPYL BETAINE | 1,5 g |
| DIETHANOLAMINE D'ACIDES GRAS | 2 g |
| DIMETHICONE COPOLYOL | 1 g |
| POLYMERE POLYGLYCOL POLYAMINE | 1 g |
| PARFUM | QS |
| EAU PURIFIEE QSP | 100 ml |

| Exemple C : SHAMPOOING | |
|---|---|
| EXTRAIT DE MYRTE | 5 g |
| ZINC PYRITHIONE PUR | 1 g |
| ACETAMIDE MEA | 0,5 g |
| ALKYL POLYGLUCOSIDE | 7 g |
| LAURYLETHERSULFATE DE SOUDE | 15 g |
| LIPOPROTEINES DE BLE | 8 g |
| LAURYL AMIDE BETAINE | 2 g |
| GLUTAMATE DOE/20 | 3 g |
| GOMME XANTHANE | 0,4 g |
| DIETHANOLAMIDE D'ACIDES GRAS | l g |
| ESSENCE DE GIROFLE | 0,05 g |
| EAU PURIFIEE QSP | 100 ml |

| Exemple D : LOTION ANTIPELLICULAIRE | |
|---|---|
| EXTRAIT DE MYRTE | 0,5 g |
| PIROCTONE-OLAMINE | 0,02 g |
| DIMETHICONE COPOLYOL | 0,20 g |
| EXTRAIT DE CAPUCINE | 0, 50 g |
| ALCOOL 95 % | 53 g |
| PARFUM          QS | |
| EAU PURIFIEE          QSP | 100 ml |

| Exemple E - BAUME RINCE ANTIPELLICULAIRE | |
|---|---|
| EXTRAIT DE MYRTE | 5 g |
| UNDECYLINATE DE ZINC | 1 g |
| ACIDE SALICYLIQUE | 0,5 g |
| STEARAMINE OXYDE | 3 g |
| LAURAMINE OXYDE | 1 g |
| ACIDE CHLORHYDRIQUE ET | |
| CHLORURE DE POTASSIUM | QS |
| LACTAMIDE MEA | 0,50 g |
| PARFUM | QS |
| EAU PURIFIEE | 100 ml |

| Légende de la figure unique | | |
|---|---|---|
| (1) | Extrait de myrte | 0,3 % |
| (2) | Undécyl = undécylényl sulfosuccinate de sodium | 2 % |
| (3) | Association (myrte + undécyl) | 0,3 % + 2 % |

**Revendications**

1. Composition dermatologique et/ou cosmétologique, **caractérisée en ce qu'**elle contient un extrait de Myrte associé à un agent anti-fongique choisi parmi le zinc pyrithione, les dérivés de l'acide undécylénique et en particulier les undécylényl sulfosuccinates, notamment les undécylényl sulfosuccinates de sodium et de zinc.

2. Composition selon la revendication 1, **caractérisée en ce qu'**elle contient un extrait de Myrte et du zinc pyrithione dans un rapport pondéral compris entre 1 et 1000.

3. Composition selon la revendication 1, **caractérisée en ce qu'**elle contient un extrait de Myrte et de l'undécylényl sulfosuccinate de sodium dans un rapport pondéral compris entre 1 et 3.

4. Composition selon la revendication 3, **caractérisée en ce qu'**elle répond à la formulation suivante :

| Extrait de Myrte | 3 - 5 g |
|---|---|
| Undécylényl sulfosuccinate de sodium | 1 - 2 g |
| Acide salicylique | 0,5 - 2 g |
| Alkyléther sulfate de sodium | 10 - 15 g |
| Cocamidopropyl bétaïne | 1 - 5 g |
| Diéthanolamine d'acides          QSP | 100 ml |

5. Utilisation d'une composition selon l'une des revendications 1 à 4, ou d'une composition comprenant un extrait de Myrte associé à la Piroctone olamine pour la fabrication d'un médicament contre le Pityrosporum ovale, responsable des pellicules.

## Claims

1. Dermatological and/or cosmetic composition, **characterized in that** it contains a myrtle extract combined with an antifungal agent chosen from zinc pyrithione, undecylenic acid derivatives and in particular undecylenyl sulphosuccinates, in particular sodium and zinc undecylenyl sulphosuccinates.

2. Composition according to Claim 1, **characterized in that** it contains a myrtle extract and zinc pyrithione in a weight ratio of between 1 and 1 000.

3. Composition according to Claim 1, **characterized in that** it contains a myrtle extract and sodium undecylenyl sulphosuccinate in a weight ratio of between 1 and 3.

4. Composition according to Claim 3, **characterized in that** it corresponds to the following formula:

| myrtle extract | 3-5 g |
|---|---|
| sodium undecylenyl sulphosuccinate | 1-2 g |
| salicylic acid | 0.5-2 g |
| sodium alkyl ether sulphate | 10-15 g |
| cocamidopropylbetaine | 1-5 g |
| diethanolamine of acids         qs | 100 ml |

5. Use of a composition according to one of Claims 1 to 4, or of a composition comprising a myrtle extract combined with Piroctone olamine for the manufacture of a medicament against Pityrosporum ovale, which is responsible for dandruff.

## Patentansprüche

1. Dermatologische und/oder kosmetische Zusammensetzung, **dadurch gekennzeichnet, daß** sie einen Extrakt von Myrte in Verbindung mit einem Antifungusmittel enthält, das ausgewählt ist aus Zinkpyrithion, Derivaten von Undecylensäure und speziell Undecylenylsulfosuccinaten, insbesondere Natrium- und Zinkundecylenylsulfosuccinat.

2. Zusammensetzung nach Anspruch 1, **dadurch gekennzeichnet, daß** sie einen Extrakt von Myrte und Zinkpyrithion in einem Gewichtsverhältnis von 1 bis 1000 enthält.

3. Zusammensetzung nach Anspruch 1, **dadurch gekennzeichnet, daß** sie einen Extrakt von Myrte und Natriumundecylenylsulfosucccinat in einem Gewichtsverhältnis von 1 bis 3 enthält.

4. Zusammensetzung nach Anspruch 3, **dadurch gekennzeichnet, daß** sie der folgenden Formulierung entspricht:

| Extrakt von Myrte | 3 - 5 g |
|---|---|
| Natriumundecylenylsulfosuccinat | 1 - 2 g |
| Salicylsäure | 0,5 - 2 g |
| Natriumalkylethersulfat | 10 - 15 g |
| Cocamidopropylbetain | 1 - 5 g |
| Diethanolamin von Säuren q.s.p. | 100 ml |

5. Verwendung einer Zusammensetzung nach einem der Ansprüche 1 bis 4 oder einer Zusammensetzung, die einen Extrakt von Myrte in Verbindung mit Pirocton Olamin umfaßt, für die Herstellung eines Medikaments gegen Pityrosporum ovale, das für Schuppen verantwortlich ist,

FIGURE UNIQUE